(19) Europäisches Patentamt
European Patent Office
Office européen des brevets

(11) EP 4 091 641 A1

(12) **EUROPEAN PATENT APPLICATION**
published in accordance with Art. 153(4) EPC

(43) Date of publication:
**23.11.2022 Bulletin 2022/47**

(21) Application number: **21740983.8**

(22) Date of filing: **12.01.2021**

(51) International Patent Classification (IPC):
***A61L 27/34*** (2006.01) ***A61F 2/30*** (2006.01)
***A61L 27/04*** (2006.01) ***A61L 27/10*** (2006.01)
***A61L 27/14*** (2006.01) ***A61L 27/16*** (2006.01)
***A61L 27/18*** (2006.01) ***A61L 27/32*** (2006.01)
***A61L 27/36*** (2006.01) ***A61L 27/50*** (2006.01)

(52) Cooperative Patent Classification (CPC):
**A61F 2/30; A61L 27/04; A61L 27/10; A61L 27/14; A61L 27/16; A61L 27/18; A61L 27/32; A61L 27/34; A61L 27/36; A61L 27/50**

(86) International application number:
**PCT/JP2021/000620**

(87) International publication number:
**WO 2021/145298 (22.07.2021 Gazette 2021/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.01.2020 JP 2020005371**

(71) Applicant: **Kyocera Corporation**
**Kyoto-shi Kyoto 612-8501 (JP)**

(72) Inventors:
- **YAMANE, Shihori**
**Kyoto-shi, Kyoto 612-8501 (JP)**
- **KYOMOTO, Masayuki**
**Kyoto-shi, Kyoto 612-8501 (JP)**
- **NOGUCHI, Aya**
**Kyoto-shi, Kyoto 612-8501 (JP)**
- **MASHIKO, Takako**
**Kyoto-shi, Kyoto 612-8501 (JP)**

(74) Representative: **TBK**
**Bavariaring 4-6**
**80336 München (DE)**

# (54) METHOD FOR PRODUCING ARTIFICIAL JOINT SLIDING MEMBER, ARTIFICIAL JOINT SLIDING MEMBER, AND ARTIFICIAL JOINT

(57) A manufacturing method of a sliding member for an artificial joint according to the present disclosure includes exposing a base member with ultraviolet rays in a state where the base member is in contact with an aqueous treatment solution containing a compound having 0.20 mol/L or more and less than 0.50 mol/L of phosphorylcholine group and a water-soluble inorganic salt.

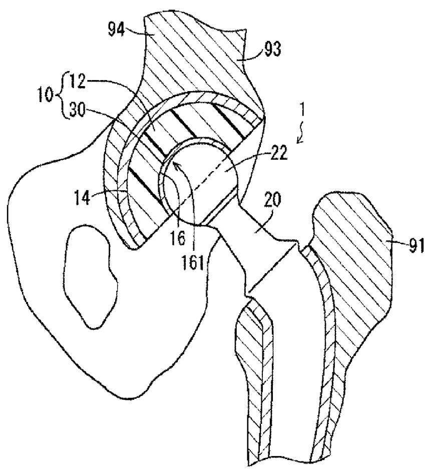


FIG. 1

EP 4 091 641 A1

Processed by Luminess, 75001 PARIS (FR)

## Description

### Technical Field

The present disclosure relates to a manufacturing method of a sliding member for an artificial joint, a sliding member for an artificial joint, and an artificial joint.

### Background Art

There has been established a treatment method in which a joint that has lost its original function due to trauma or disease such as osteoarthritis is replaced with an artificial joint. A known sliding material used for such an artificial joint includes an artificial joint member whose sliding surface is made of a polymer having a phosphorylcholine group.

### Summary

### Technical Problem

One aspect of the present disclosure provides a sliding member for an artificial joint having wear resistance equal to or higher than that of the related art while reducing a concentration of a compound having a phosphorylcholine group.

### Solution to Problem

A manufacturing method of a sliding member for an artificial joint according to the present disclosure includes exposing a base member with ultraviolet rays in a state where the base member is in contact with an aqueous treatment solution containing a compound having 0.20 mol/L or more and less than 0.50 mol/L of phosphorylcholine group and a water-soluble inorganic salt.

Further, the sliding member for an artificial joint according to the present disclosure includes a base member including a compound having a methylene group and a polymer film that is formed on at least a portion of a surface of the base member and includes a polymer chain in which a compound having a phosphorylcholine group is polymerized, and the following formula (1) is satisfied:

$$\text{(peak intensity of phosphoric acid group/peak intensity of methylene group in infrared spectral analysis spectrum)/film thickness of polymer film} \geq 0.007\ \text{nm}^{-1} \ldots (1).$$

Alternatively, the sliding member for an artificial joint according to the present disclosure includes a base member including a compound having a benzene ring and a polymer film that is formed on at least a portion of a surface of the base member and includes a polymer chain in which a compound having an ester group and a phosphorylcholine group is polymerized, and the following formula (2) is satisfied:

$$\text{(peak intensity of ester group/peak intensity of benzene ring in infrared spectral analysis spectrum)/film thickness of polymer film} \geq 0.0005\ \text{nm}^{-1} \ldots (2).$$

### Advantageous Effects of Invention

One aspect of the present disclosure allows for providing the sliding member for an artificial joint having wear resistance that can be used for a member for an artificial joint member while reducing the concentration of a compound having a phosphorylcholine group.

### Brief Description of Drawings

FIG. 1 is a schematic view of an artificial hip joint 1 according to one embodiment.

FIG. 2 is a schematic view of an acetabular cup 10 according to one embodiment.

FIG. 3 is a diagram illustrating a result of simulation in Reference Example 1.

FIG. 4 is a graph showing a relationship between a concentration of 2-methacryloyloxyethyl phosphorylcholine in an aqueous treatment solution and a density (phosphoric acid index/film thickness) of a formed polymer film in examples and comparative examples using UHMWPE as a base member.

FIG. 5 is a graph showing a relationship between the concentration of 2-methacryloyloxyethyl phosphorylcholine in an aqueous treatment solution and a weight wear rate of a formed polymer film in examples and comparative examples using UHMWPE as a base member.

FIG. 6 is a graph showing a relationship between the concentration of 2-methacryloyloxyethyl phosphorylcholine in an aqueous treatment solution and a density (ester index/film thickness) of a formed polymer film in examples and comparative examples using PEEK as a base member.

Description of Embodiments

Hereinafter, one embodiment will be described in detail. Unless otherwise specified in the present specification, "A to B" representing a numerical value range means "A or more and B or less".

1. Manufacturing Method of Sliding Member for Artificial Joint

The manufacturing method of a sliding member for an artificial joint according to one embodiment includes a step of exposing a base member with ultraviolet rays in a state where the base member is in contact with an aqueous treatment solution containing a compound having 0.20 mol/L or more and less than 0.50 mol/L of phosphorylcholine group and a water-soluble inorganic salt. This allows a polymer film including a polymer chain in which a compound having a phosphorylcholine group is polymerized to be formed on at least a portion of a surface of the base member. That is, the sliding member for an artificial joint in which the surface of the base member is covered with the polymer film may be obtained. Hereinafter, the compound having a phosphorylcholine group is also referred to as a "PC compound".

An artificial joint, which is implanted in a living body, is necessary to be safe and maintain a constant function in the living body for a long period of time. Avoiding replacing the artificial joint with a new one as much as possible allows the physical or economic burden on the patient to be reduced.

For example, a total replacement artificial joint includes mainly two members, and each of the two members is mounted on an end portion of the bone. When a joint is moved, these two members move and slide relatively. As repeatedly slid, these members may generate wear powder. Since the generated wear powder is recognized as a foreign matter in the living body, the biological immune system functions to eliminate the foreign matter. At this time, multinucleated cells called osteoclasts are activated, causing osteolysis in which the bone around the artificial joint is resorbed. This osteolysis may cause a void between the bone and the artificial joint, generating the loosening of the artificial joint. The artificial joint inevitably slides, and a sliding material that suppresses generation of such wear powder and has excellent wear resistance is required.

On the other hand, the PC compound is expensive, and reducing the usage of the PC compound is awaited from the viewpoint of production cost. Also, in treating the artificial joint member using the PC compound, not all of the compounds used are bonded to the surface of the artificial joint member. Since the unbonded PC compound will be discarded, the usage thereof is preferably small from the viewpoint of the environmental impact. The related art as described above has room for enhancement from the viewpoint of implementing a sliding member for an artificial joint having wear resistance equal to or higher than that of the related art while reducing the concentration of the PC compound.

As a result of intensive studies, the present inventor has found that using the PC compound together with a water-soluble inorganic salt at a specific concentration allows the sliding member for an artificial joint having wear resistance equal to or higher than that of the related art to be implemented though the concentration of the PC compound is lower than that of the related art.

The present inventor has found that a water-soluble inorganic salt is added to an aqueous treatment solution containing a PC compound to improve radical polymerization efficiency, thus enabling a polymer film to be formed on a surface of a base member by using an aqueous treatment solution having a concentration lower than that of the related art. Further, the present inventor has found from the results of simulations based on molecular orbital calculations and actual tests that a lower concentration of the aqueous treatment solution is not necessarily better and that there is a suitable concentration range.

Furthermore, a high-density polymer film can be formed by using an aqueous treatment solution containing the PC compound at the specific concentration described above together with the water-soluble inorganic salt. This allows the sliding member for an artificial joint having wear resistance equal to or higher than that of the related art to be implemented.

1-1. Polymer Film Forming Step

In the present specification, the step of exposing a base member with ultraviolet rays in a state where the base member is in contact with an aqueous treatment solution is also referred to as a "polymer film forming step".

A cartilage surface of the joint of the living body is covered with the phospholipid. This phospholipid contributes to protection and lubrication of cartilage. A polymer film including a polymer chain in which a PC compound having a chemical structure close to that of this phospholipid is polymerized forms a sliding surface, maintaining a good lubrication state for a long period of time. As a result, a sliding member for an artificial joint which is subject to extremely low wear and has an impact absorbing function can be obtained.

The aqueous treatment solution described above contains the PC compound and a water-soluble inorganic salt in addition to water. The concentration of the PC compound in the aqueous treatment solution is 0.20 mol/L or more and less than 0.50 mol/L, may be 0.20 mol/L or more and 0.40 mol/L or less, may be 0.20 mol/L or more and 0.33 mol/L or less, may be 0.20 mol/L or more and 0.30 mol/L or less, or may be 0.225 mol/L or more and 0.30 mol/L or less. The above concentration allows the production costs and the environmental impact to be reduced, a polymer film having sufficient density to be formed, and excellent wear resistance to be provided to the sliding member for an artificial joint.

Selecting, as the PC compound, in particular, a polymerizable monomer having a phosphorylcholine group at one end and a functional group graft-polymerizable with the polymer base member at the other end allows the polymer film to be graft-bonded to the sliding surface of the base member.

Examples of the PC compound include 2-methacryloyloxyethyl phosphorylcholine, 2-acryloyloxyethyl phosphorylcholine, 4-methacryloyloxybutyl phosphorylcholine, 6-methacryloyloxyhexyl phosphorylcholine, and ω-methacryloyloxyethylene phosphorylcholine. Of these, 2-methacryloyloxyethyl phosphorylcholine may be used. Hereinafter, 2-methacryloyloxyethyl phosphorylcholine is also referred to as "MPC". A polymer in which MPC is polymerized is also referred to as poly (MPC) or PMPC.

MPC, which has a chemical structure represented by the following structural formula, is a polymerizable monomer having a phosphorylcholine group and a polymerizable methacrylic acid unit.

[Chem. 1]

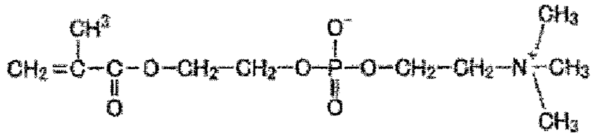

MPC can be easily polymerized by radical polymerization, forming a high molecular weight homopolymer (Ishihara et al., Polymer Journal 22, p355 (1990)). Therefore, forming a polymer film as an aggregate of polymer chains in which MPC is polymerized allows graft-bonding between the MPC polymer chains and the sliding surface of the base member to be performed under relatively mild conditions. Further, forming a high-density polymer film allows a large number of phosphorylcholine groups to be formed on the sliding surface of the base member.

The polymer film described above can be formed not only as a homopolymer composed of a single polymerizable monomer having a phosphorylcholine group but also as a copolymer composed of a polymerizable monomer having a phosphorylcholine group and, for example, another vinyl compound monomer. This enables a function for improving mechanical strength and the like to be added to the polymer film depending on the type of other vinyl compound monomers used.

Examples of the water-soluble inorganic salt described above include an alkali metal salt, and an alkaline earth metal salt. Examples of the alkali metal salt include a sodium salt, a potassium salt, a lithium salt, and a cesium salt. Examples of the alkaline earth metal salt include a magnesium salt, a calcium salt, a strontium salt, a barium salt, and a radium salt. Further, examples of the water-soluble inorganic salt classified according to the type of counter anion include halides (for example, chloride, fluoride, bromide, and iodide), phosphoric acids, carbonates, nitrates, and hydroxides. The water-soluble inorganic salt is one or more selected from the group consisting of, for example, sodium chloride, potassium chloride, calcium chloride, and magnesium chloride.

The concentration of the water-soluble inorganic salt in the aqueous treatment solution can be set to, for example, 0.01 to 5.0 mol/L. Furthermore, the concentration of the water-soluble inorganic salt in the aqueous treatment solution can be set to, for example, 1.0 to 5.0 mol/L, or 1.0 to 3.0 mol/L. The above concentration allows a polymer film having sufficient graft density to be efficiently formed.

For example, according to one embodiment, by using the water-soluble inorganic salt, a polymer film having a film thickness of 100 nm or more can be formed on the surface of the base member in a short time of 1 to 90 minutes. More specifically, a polymer film having a film thickness of 600 nm or more can also be obtained. Even with such a large film thickness, no voids, etc., are observed at the interface between the film and the base member, and it can be expected

to have sufficient film strength.

Examples of the material constituting the base member include polymeric materials such as polyolefin-based materials such as polyethylene, and aromatic polyether ketone-based materials, such as polyether ether ketone (PEEK). These polymeric materials may include functional compounds, such as antioxidants and crosslinking agents, and/or reinforcing materials, such as carbon fibers.

Examples of the polyethylene include ultra-high molecular weight polyethylene (UHMWPE). The UHMWPE, which has excellent mechanical characteristics such as wear resistance, impact resistance, and deformation resistance, can be used for artificial joints as a resin material. In addition, polyether ether ketone (PEEK) also has excellent mechanical characteristics such as impact resistance, and deformation resistance, and thus can be used in artificial joints as resin materials.

The higher the molecular weight of the polymer constituting the base member is, the higher the wear resistance tends to be. When the base member includes a compound having a methylene group such as polyethylene, the molecular weight of the polymer constituting the base member may be 1 million or more, 1 million to 7 million, 3 million to 7 million, or 3 million to 4 million. When the base member includes a compound having a benzene ring such as the PEEK, the molecular weight of the polymer constituting the base member may be 50,000 or more, 80,000 to 500,000, or 80,000 to 200,000. In the present specification, the molecular weight of the polymer constituting the base member means the molecular weight determined by the following formula (3) by measuring the viscosity of a decahydronaphthalene (decalin) solution containing the polymer at 135°C.

$$\text{Molecular weight} = 5.37 \text{ x } 104 \text{ x (intrinsic viscosity) } 1.49 \ldots(3)$$

From the viewpoint of mechanical characteristics such as the impact resistance and the deformation resistance, the density of the polymer constituting the base member may be 0.927 to 0.944 g/cm$^3$ when the base member includes the compound having a methylene group. Also, when the base member includes the compound having a benzene ring, the density may be 1.20 to 1.55 g/cm$^3$.

The polymer film may be formed at a portion corresponding to a sliding surface that is at least a portion of the surface of the base member. For example, in manufacturing an acetabular cup in an artificial hip joint, the polymer film may be formed on an inner spherical surface of the cup with which at least the femoral head comes into contact.

The polymer film may be formed by photo-initiated graft polymerization of a polymer chain in which the PC compound is polymerized with the sliding surface of the base member. The photo-initiated graft polymerization can stably immobilize the polymer chain in which the PC compound is polymerized on the surface of the base member. Further, the photo-initiated graft polymerization forms a polymer chain having a phosphorylcholine group at a high density on the sliding surface of the base member, enabling the density of the polymer film to be increased.

To perform the photo-initiated graft polymerization of the polymer chain with the sliding surface of the base member, the sliding surface of the base member is exposed with ultraviolet rays in a state where the base member is in contact with an aqueous treatment solution containing the PC compound as a polymerizable monomer and the water-soluble inorganic salt. At this time, it is also possible to heat the base member. That is, the manufacturing method may further include the step of heating the base member. By heating the base member and the aqueous treatment solution in contact with the base member, the photo-initiated graft polymerization reaction can be controlled.

When the sliding surface of the base member is exposed with ultraviolet rays, the PC compound in the vicinity of the sliding surface polymerizes to produce the polymer chain. The produced polymer chain is covalently bonded to the sliding surface of the base member. Causing the polymer chain to be graft-bonded to the sliding surface at a high density forms the polymer film covering the sliding surface of the base member as a whole.

Before the base member is in contact with the aqueous treatment solution, a photopolymerization initiator may be applied to the sliding surface of the base member. In this case, a photopolymerization initiator radical generated by the ultraviolet exposure forms a polymerization initiation point on the surface of the base member. The PC compound, which is a polymerizable monomer, reacts with the polymerization initiation point to initiate graft polymerization and grow into a graft polymer.

The wavelength of the ultraviolet rays to be exposed is, for example, 300 to 400 nm. Examples of ultraviolet exposing sources that can be used include high-pressure mercury lamps (UVL 400HA, manufactured by Riko Kagaku Sangyo Co., Ltd.) and LEDs (MeV365 P601JMM, manufactured by YEV Co., Ltd.). The ultraviolet exposing time may be 11 to 90 minutes or may be 23 to 90 minutes.

Further, after the polymer film forming step, sterilization treatment by gamma irradiation can be performed.

1-2. Base Member Forming Step

The manufacturing method of a sliding member for an artificial joint according to one embodiment may include

a base member forming step of molding a polymeric material to obtain a base member before the polymer film forming step.

For example, the base member can be obtained by charging a powdery, granular, or pellet-like polymeric material into a metal mold, followed by compression molding, extrusion molding, or injection molding. Examples of the polymeric material include the UHMWPE and the PEEK described above. The UHMWPE and the PEEK, which are thermoplastic resins, have less flowability than the melting temperature. Therefore, the UHMWPE or the PEEK in a solid state may be charged into a metal mold and molded under high heat and pressure conditions. An antioxidant, a crosslinking agent, or a reinforcing material, such as carbon fiber, together with the polymeric material may be added to the metal mold.

The base member obtained by the compression molding, the extrusion molding, or the injection molding may be subjected to the subsequent polymer film forming step as it is or may be subjected to the polymer film forming step after shaping by machining.

1-3. Crosslinking Step

The manufacturing method of a sliding member for an artificial joint according to one embodiment may include a crosslinking step of generating a crosslinked structure in a molecule of the polymeric material before the polymer film forming step. This obtains a base member having further improved mechanical characteristics, such as wear resistance.

The crosslinking step may include a step of irradiating the base member with a high energy ray. This step is also referred to as a high energy ray irradiation step.

The base member including the UHMWPE is irradiated with the high energy ray to generate a free radical. This causes the UHMWPE to be bonded between molecular chains, enabling the UHMWPE having a crosslinked structure to be obtained. Generating the crosslinked structure between the molecular chains improves the mechanical characteristics, such as wear resistance and impact resistance.

The crosslinking reaction is made possible by adding a crosslinking agent, but completely removing unreacted crosslinking agent tends to be difficult. Therefore, the crosslinking reaction by high energy ray irradiation may be used in consideration of the influence of the unreacted crosslinking agent on the living body.

Examples of the high energy ray include X-rays, gamma rays, and electron beams. The irradiation dose of the high energy ray is, for example, 25 to 200 kGy but may be 50 to 150 kGy. Examples of the high energy ray source that can be used include a radiation device using Co (cobalt) 60 as a radiation source as a gamma ray source, an accelerator that emits an electron beam, and a device that emits an X-ray.

The crosslinking step may further include a thermal treatment step after the high energy ray irradiation step. In the thermal treatment step, the free radical generated by the high energy ray irradiation step is more efficiently consumed in the crosslinking reaction to promote intramolecular crosslinking. The temperature range of the thermal treatment may be 110 to 130°C. The thermal treatment time may be 2 to 12 hours.

2. Sliding Member for Artificial Joint

A sliding member for an artificial joint according to one embodiment includes a base member including a compound having a methylene group, and a polymer film that is formed on at least a portion of a surface of the base member and includes a polymer chain in which a compound having a phosphorylcholine group is polymerized, and the following formula (1) is satisfied:

$$\text{(peak intensity of phosphoric acid group/peak intensity of methylene group in infrared spectral analysis spectrum)/film thickness of polymer film} \geq 0.007\ \text{nm}^{-1} \ldots (1)$$

A sliding member for an artificial joint according to another embodiment includes a base member including a compound having a benzene ring, and a polymer film that is formed on at least a portion of a surface of the base member and includes a polymer chain in which a compound having an ester group and a phosphorylcholine group is polymerized, and the following formula (2) is satisfied:

$$\text{(peak intensity of ester group/peak intensity of benzene ring in infrared spectral analysis spectrum)/film thickness of polymer film} \geq 0.0005\ \text{nm}^{-1} \ldots (2)$$

Such a sliding member for an artificial joint can be obtained by, for example, the manufacturing method of a sliding member for an artificial joint according to the above-described one embodiment. The matters already described

in [1. Manufacturing Method of Sliding Member for Artificial Joint] will not be described below.

The polymer film is formed by a polymer chain obtained by initiating polymerization of the PC compound from the sliding surface of the base member. The present inventor has found that the wear resistance is improved as the amount of the polymer chain in which the PC compound is polymerized per film thickness of the polymer film is increased. As an index of the amount of the polymer chain, a "phosphoric acid index" or an "ester index" can be used.

Here, the "phosphoric acid index" is defined by a peak intensity of 1460 $cm^{-1}$, which corresponds to an absorption of a phosphoric acid group, with respect to a peak intensity of 1080 $cm^{-1}$, which corresponds to an absorption of a methylene group, in a spectrum of Fourier-transform infrared spectroscopy (FT-IR) analysis. That is, the phosphoric acid index is represented as peak intensity of phosphoric acid group/peak intensity of methylene group in infrared spectroscopic spectrum.

Similarly, the "ester index" is defined by a peak intensity of 1600 $cm^{-1}$, which corresponds to an absorption of an ester group, with respect to a peak intensity of 1730 $cm^{-1}$, which corresponds to an absorption of a benzene ring, in a spectrum of Fourier-transform infrared spectroscopy (FT-IR) analysis. That is, the ester index is represented as peak intensity of ester group/peak intensity of benzene ring in infrared spectroscopic spectrum.

When a polymer film including the PC compound formed on the base member including a methylene group is subjected to FT-IR measurement, a peak of the methylene group derived from the base member and a peak of a phosphoric acid group derived from the polymer film are observed. The value obtained by dividing the phosphoric acid index calculated from the peak intensity due to the absorption of the methylene group and the peak intensity due to the absorption of the phosphoric acid group by the film thickness of the polymer film is considered to be a graft chain density present per unit area of the surface of the base member.

In addition, when a polymer film including the PC compound formed on the base member including a benzene ring is subjected to the FT-IR measurement, a peak of the benzene ring derived from the base member and a peak of an ester group derived from the polymer film are observed. The value obtained by dividing the ester index calculated from the peak intensity due to the absorption of the benzene ring and the peak intensity due to the absorption of the ester group by the film thickness of the polymer film is also considered to be the graft chain density present per unit area of the surface of the base member. Even when the polymer film including the PC compound formed on the base member including the benzene ring is subjected to the FT-IR measurement, the peak of the phosphoric acid group derived from the polymer film may not be observed. Therefore, the peak of the ester group is used as an index instead of the peak of the phosphoric acid group. The compound having an ester group and a phosphorylcholine group is used as the PC compound, and the value obtained by dividing the ester index by the film thickness of the polymer film can also be regarded as corresponding to the graft chain density.

"(Peak intensity of phosphoric acid group/peak intensity of methylene group in infrared spectroscopic spectrum)/film thickness of polymer film" is also expressed as "phosphoric acid index/film thickness of polymer film". "(Peak intensity of ester group /peak intensity of benzene ring in infrared spectroscopic spectrum)/film thickness of polymer film" is also expressed as "ester index/film thickness of polymer film". The parameter represented by the "phosphoric acid index/film thickness of polymer film" or the "ester index/film thickness of polymer film" is also referred to as "density of polymer film" for convenience in the present specification.

As illustrated in examples described later, the use of the aqueous treatment solution containing the PC compound at a high concentration does not necessarily obtain a polymer film with high density and excellent wear resistance. This cannot be predicted even by a person skilled in the art. That is, a sliding member for an artificial joint satisfying Formula (1) or Formula (2) has not been easily obtained in accordance with the conventional common technical knowledge.

When the base member includes the compound having a methylene group, the density of the polymer film may be 0.007 $nm^{-1}$ or more or may be 0.008 $nm^{-1}$ or more. When the density of the polymer film is 0.007 $nm^{-1}$ or more, the polymer film exhibits wear resistance equal to or higher than that of the related art even the use of the aqueous treatment solution containing the PC compound at a concentration lower than that of the related art. The upper limit of the density of the polymer film is not particularly limited but may be, for example, 0.020 $nm^{-1}$ or less, may be 0.015 $nm^{-1}$ or less, or may be 0.012 $nm^{-1}$ or less.

When the base member includes the compound having a benzene ring, the density of the polymer film may be 0.0005 $nm^{-1}$ or more or may be 0.0008 $nm^{-1}$ or more. When the density of the polymer film is 0.0005 $nm^{-1}$ or more, the polymer film exhibits wear resistance equal to or higher than that of the related art even the use of the aqueous treatment solution containing the PC compound at a concentration lower than that of the related art. The upper limit of the density of the polymer film is not particularly limited but may be, for example, 0.0020 $nm^{-1}$ or less, may be 0.0015 $nm^{-1}$ or less, or may be 0.0012 $nm^{-1}$ or less.

The film thickness of the polymer film may be 50 to 1000 nm, may be 100 nm or more, or may be 100 to 500 nm from the viewpoint of obtaining sufficient wear resistance.

The base member includes compounds having at least a methylene group or a benzene ring. Examples of these compounds include polyethylene or the PEEK. In particular, the UHMWPE may be used as described in [1. Manufacturing Method of Sliding Member for Artificial Joint]. In addition, the molecular weight and density of the compound

having a methylene group may be the same as the molecular weight and density of the polymer constituting the base member described in [1. Manufacturing Method of Sliding Member for Artificial Joint].

The artificial joint to which the above-described sliding member for an artificial joint is applied is not particularly limited. Examples of the artificial joint include an artificial hip joint, an artificial knee joint, an artificial ankle joint, an artificial shoulder joint, an artificial elbow joint, an artificial finger joint, and an artificial intervertebral disc. For example, the artificial hip joint may include a femoral head and an acetabulum. The sliding member for an artificial joint according to one embodiment can be applied to the femoral head or the acetabulum, or both. For example, the sliding member for an artificial joint according to one embodiment may be used for either one of the femoral head and the acetabulum. In this case, a member including a metal such as stainless steel or a cobalt chromium alloy, a ceramic such as alumina or zirconia, a polymer such as the UHMWPE or the PEEK may be used for the other. Also, for example, the femoral head and acetabulum may be formed of different materials. For example, the femoral head may be formed of a polymer, ceramic, or metal material, and the base member of the acetabulum may be formed of, for example, a polymeric material.

Hereinafter, an example in which the sliding member for an artificial joint according to one embodiment is used as an acetabular cup of an artificial hip joint will be described. FIG. 1 is a schematic view of an artificial hip joint 1 according to one embodiment. FIG. 2 is a schematic view of an acetabular cup 10 according to one embodiment. The artificial hip joint 1 is composed of the acetabular cup 10 to be fixed to an acetabulum 94 of a hip bone 93 and a femoral stem 20 to be fixed to a proximal end of a femur 91. The acetabular cup 10 includes a cup base member 12 having a substantially hemispherical acetabular fixing surface 14 and a substantially hemispherically recessed sliding surface 16, and a polymer film 30 covering the sliding surface 16. A femoral head 22 of the femoral stem 20 fitted into a recess 161 in which the polymer film 30 of the acetabular cup 10 is formed is slid to function as a hip joint. The acetabular fixing surface 14 is an outer surface disposed closer to the acetabulum 94. The sliding surface 16 is also an inner surface or contact surface (first contact surface) configured to contact the femoral head 22.

As illustrated in FIGS. 1 and 2, in the acetabular cup 10, the sliding surface 16 of the cup base member 12 is covered with the polymer film 30. The polymer film 30 is obtained by graft-bonding a polymer chain in which the PC compound is polymerized with the sliding surface 16. The polymer film 30 may be disposed only at the acetabular cup 10 and may be disposed on both the acetabular cup 10 and the femoral head 22.

Also, the surface of the acetabular cup 10 may have a first region and a second region different from the first region. For example, the film thickness of the polymer film 30 in the second region is thicker than that in the first region. Furthermore, the polymer film 30 may be disposed only on the sliding surface 16 of the acetabular cup 10. In this case, the sliding surface 16 is the second region, and the surface other than the sliding surface 16 of the acetabular cup 10 can be regarded as the first region. The first region is located on the outer surface of the surfaces of the acetabular cup 10, and it can also be said that the second region is located on the inner surface configured to contact the femoral head 22 of the surfaces of the acetabular cup 10. Further, the first region is located on an edge portion located between the outer surface and the inner surface of the surfaces of the acetabular cup 10, and the second region may be located on the inner surface of the surfaces of the acetabular cup 10.

Also, the acetabular cup 10 has the first contact surface configured to contact the femoral head 22, and the femoral head 22 may have a second contact surface that is configured to contact the acetabular cup 10 and has a surface roughness less than the first contact surface. The surface roughness of the second contact surface has an average roughness Ra of, for example, 0.1 microns or less.

The polymer film 30 has a structure similar to that of a biological film, has a high affinity with lubricating fluid in the joint, and can retain the lubricating fluid inside the film. Furthermore, the polymer film 30 includes a phosphoric acid group at a high density. Thus, the acetabular cup 10 exhibits superior wear resistance.

The invention according to the present disclosure has been described above based on the drawings and examples. However, the invention according to the present disclosure is not limited to each embodiment described above. That is, the invention according to the present disclosure can be variously modified within the scope indicated in the present disclosure, and an embodiment to be obtained by appropriately combining technical means disclosed in different embodiments is also included in the technical scope of the invention according to the present disclosure. In other words, it should be noted that a person skilled in the art can easily make various variations or modifications based on the present disclosure. It should also be noted that these variations or modifications are included within the scope of the present disclosure.

## Examples

Hereinafter, the invention according to the present disclosure will be described in more detail based on reference examples, examples, and comparative examples, but the invention according to the present disclosure is not limited to these examples.

Reference Example 1 (Simulation)

As described above, the polymer film including the polymer chain in which the PC compound is polymerized can be formed by exposing the base member with ultraviolet rays in a state where the base member is in contact with the aqueous treatment solution containing the PC compound together with a water-soluble inorganic salt at a specific concentration.

First, a simulation was performed on the assumption of using sodium chloride as the water-soluble inorganic salt and MPC as the PC compound. Specifically, the distance between the double bond portions of the methacrylic group that contributes to the polymerization of two MPC molecules was evaluated by simulation calculation in a case where the sodium chloride concentration in the aqueous treatment solution was 2.5 mol/L and where the MPC concentration was changed from 0.00 mol/L to 0.50 mol/L. Amber was used as simulation software, and the following conditions were set. The General AMBER Force Field (GAFF) and electrostatic potential (RESP) charge (calculated by HF/6-31G*) were used as a force field of solute, and the TIP3P was used as a force field of water molecules. A constraint condition by the SHAKE method was imposed, and the particle mesh Ewald (PME) method was used for long-range electrostatic interactions. Under the above conditions, the molecular dynamics simulations in the Langevin heat bath at 300 K were performed.

The results are illustrated in FIG. 3. The vertical axis of FIG. 3 represents a radial distribution function. In the simulation, the radial distribution function represents the frequency at which the distance between the double bond portions of the two MPC molecules becomes shortest at each MPC concentration. From FIG. 3, it was found that the frequency at which the distance between the double bond portions of the two MPC molecules became shortest increased when the MPC concentration was 0.20 mol/L or more and less than 0.50 mol/L. From this, it was expected that a sliding member for an artificial joint having excellent wear resistance could be efficiently obtained by using an aqueous treatment solution having a specific MPC concentration.

Comparative Example 1

A square material (cross section: 10 mm x 3 mm, length: 100 mm) made of the UHMWPE having a molecular weight of 3.5 million and a density of 0.930 g/cm$^3$ was used as a base member. The square material was immersed in an acetone solution containing 1.0 g/dL of benzophenone for 30 seconds. Immediately thereafter, the square material was pulled up and the solvent was removed at room temperature, whereby benzophenone was sufficiently adsorbed on the square material.

The aqueous treatment solution containing 2.5 mol/L of sodium chloride was held in advance at 60°C, and then sufficiently degassed. The above-described benzophenone-adsorbed square material was immersed in the aqueous treatment solution, and then, while heating the square material, the 10 mm x 100 mm surface of the six surfaces was exposed with ultraviolet rays having wavelengths of 300 to 400 nm and an intensity of 5 mW/cm$^2$ for 90 minutes. The square material was taken out from the aqueous treatment solution and then sufficiently washed with pure water and ethanol to obtain a test piece.

Comparative Example 2

A test piece was obtained by the same method as in Comparative Example 1 except that the aqueous treatment solution containing 2.5 mol/L of sodium chloride and 0.10 mol/L of MPC was used as the aqueous treatment solution. In the test piece, a polymer film made of PMPC was formed on the 10 mm x 100 mm surface of the six surfaces of the square material.

Comparative Example 3

By the same method as in Comparative Example 2 except that the MPC concentration in the aqueous treatment solution was changed to 0.17 mol/L, a test piece in which a polymer film made of PMPC was formed on the 10 mm x 100 mm surface of the six surfaces of the square material was obtained.

Example 1

By the same method as in Comparative Example 2 except that the MPC concentration in the aqueous treatment solution was changed to 0.20 mol/L, a test piece in which a polymer film made of PMPC was formed on the 10 mm x 100 mm surface of the six surfaces of the square material was obtained.

Example 2

By the same method as in Comparative Example 2 except that the MPC concentration in the aqueous treatment solution was changed to 0.225 mol/L, a test piece in which a polymer film made of PMPC was formed on the 10 mm x 100 mm surface of the six surfaces of the square material was obtained.

Example 3

By the same method as in Comparative Example 2 except that the MPC concentration in the aqueous treatment solution was changed to 0.25 mol/L, a test piece in which a polymer film made of PMPC was formed on the 10 mm x 100 mm surface of the six surfaces of the square material was obtained.

Example 4

By the same method as in Comparative Example 2 except that the MPC concentration in the aqueous treatment solution was changed to 0.30 mol/L, a test piece in which a polymer film made of PMPC was formed on the 10 mm x 100 mm surface of the six surfaces of the square material was obtained.

Example 5

By the same method as in Comparative Example 2 except that the MPC concentration in the aqueous treatment solution was changed to 0.33 mol/L, a test piece in which a polymer film made of PMPC was formed on the 10 mm x 100 mm surface of the six surfaces of the square material was obtained.

Comparative Example 4

By the same method as in Comparative Example 2 except that the MPC concentration in the aqueous treatment solution was changed to 0.50 mol/L, a test piece in which a polymer film made of PMPC was formed on the 10 mm x 100 mm surface of the six surfaces of the square material was obtained.

Comparative Example 5

A square material (cross section: 10 mm x 3 mm, length: 100 mm) made of the PEEK having a density of 1.26 to 1.36 g/cm$^3$ was used as a base member.

The aqueous treatment solution containing 2.5 mol/L of sodium chloride was held in advance at 60°C, and then sufficiently degassed. The square material was immersed in the aqueous treatment solution, and then, while heating the square material, the 10 mm x 100 mm surface of the six surfaces was exposed with ultraviolet rays having wavelengths of 300 to 400 nm and an intensity of 5 mW/cm$^2$ for 90 minutes. The square material was taken out from the aqueous treatment solution and then sufficiently washed with pure water and ethanol to obtain a test piece.

Comparative Example 6

A test piece was obtained by the same method as in Comparative Example 5 except that the aqueous treatment solution containing 2.5 mol/L of sodium chloride and 0.10 mol/L of MPC was used as the aqueous treatment solution. In the test piece, a polymer film made of PMPC was formed on the 10 mm x 100 mm surface of the six surfaces of the square material.

Comparative Example 7

By the same method as in Comparative Example 6 except that the MPC concentration in the aqueous treatment solution was changed to 0.17 mol/L, a test piece in which a polymer film made of PMPC was formed on the 10 mm x 100 mm surface of the six surfaces of the square material was obtained.

Example 6

By the same method as in Comparative Example 6 except that the MPC concentration in the aqueous treatment solution was changed to 0.20 mol/L, a test piece in which a polymer film made of PMPC was formed on the 10 mm x 100 mm surface of the six surfaces of the square material was obtained.

Example 7

By the same method as in Comparative Example 6 except that the MPC concentration in the aqueous treatment solution was changed to 0.225 mol/L, a test piece in which a polymer film made of PMPC was formed on the 10 mm x 100 mm surface of the six surfaces of the square material was obtained.

Example 8

By the same method as in Comparative Example 6 except that the MPC concentration in the aqueous treatment solution was changed to 0.25 mol/L, a test piece in which a polymer film made of PMPC was formed on the 10 mm x 100 mm surface of the six surfaces of the square material was obtained.

Example 9

By the same method as in Comparative Example 6 except that the MPC concentration in the aqueous treatment solution was changed to 0.30 mol/L, a test piece in which a polymer film made of PMPC was formed on the 10 mm x 100 mm surface of the six surfaces of the square material was obtained.

Example 10

By the same method as in Comparative Example 6 except that the MPC concentration in the aqueous treatment solution was changed to 0.33 mol/L, a test piece in which a polymer film made of PMPC was formed on the 10 mm x 100 mm surface of the six surfaces of the square material was obtained.

Comparative Example 8

By the same method as in Comparative Example 6 except that the MPC concentration in the aqueous treatment solution was changed to 0.50 mol/L, a test piece in which a polymer film made of PMPC was formed on the 10 mm x 100 mm surface of the six surfaces of the square material was obtained.

Evaluation Method

(a) Density of Polymer Film

First, the test piece was subjected to FT-IR measurement. The FT-IR measurement was performed using an FT-IR apparatus (FT/IR-6300 type A manufactured by JASCO Corporation) with a resolution of 4 $cm^{-1}$ and a cumulative number of 64.

The test piece was also embedded in epoxy resin and then stained with ruthenium tetrachloride. Thereafter, an ultra-thin piece was cut from the test piece using an ultramicrotome. An electron microscopic image of a cut surface of the ultra-thin piece was obtained using a transmission electron microscope (JEM-1010 manufactured by JEOL Ltd.) with an accelerating voltage 100 kV. With respect to one image of the obtained electron microscopic image, the film thickness on the cut surface was measured at 10 points, and the arithmetic average value thereof was calculated as the film thickness of the polymer film.

In the case where the UHMWPE was used as the base member, the density of the polymer film was calculated based on the following formula from the peak intensity of the phosphoric acid group and the peak intensity of the methylene group in the obtained infrared spectroscopic spectrum and the film thickness of the polymer film.

density of polymer film = (peak intensity of phosphoric acid group/peak intensity of methylene group in infrared spectral analysis spectrum)/film thickness of polymer film

In the case where the PEEK was used as the base member, the density of the polymer film was calculated based on the following formula from the peak intensity of the ester group and the peak intensity of the benzene ring in the obtained infrared spectroscopic spectrum and the film thickness of the polymer film.

density of polymer film = (peak intensity of ester group/peak intensity of benzene ring in infrared spectral analysis spectrum)/film thickness of polymer film

(b) Weight Wear Rate

Weight wear rate was measured by a pin-on-disk test. Specifically, the weight wear rate was measured by a multi-directional sliding test using a pin-on-disk type wear tester (Ortho-POD, available from AMTI Inc.), with reference to ASTM F732-00 standard. The multi-directional sliding test was performed in bovine serum at 37°C. The maximum load was set to 213 N, and the test was performed up to one million cycles under conditions of a sliding length of 30 mm and a sliding speed of 1 Hz. The lubricant was replaced every 250,000 cycles, and at the same time, the test piece was collected, washed, dried, and weighed. In addition, the test piece of the control group was immersed in the lubricating liquid, and the amount of wear was calculated by correcting the amount of water absorption from the change in weight.

Evaluation Result

FIG. 4 is a graph showing the relationship between the concentration of MPC in an aqueous treatment solution and the density (phosphoric acid index/film thickness) of a formed polymer film in examples and comparative examples using the UHMWPE as a base member. FIG. 4 indicates that a high-density PMPC layer can be obtained when an aqueous treatment solution containing 0.20 mol/L or more and less than 0.50 mol/L of MPC and a water-soluble inorganic salt is used. In particular, when an aqueous treatment solution containing 0.20 mol/L or more and 0.30 mol/L or less of MPC and a water-soluble inorganic salt is used, a polymer film having a density of 0.007 $nm^{-1}$ or more can be obtained. Compared to this, for example, when the MPC concentration is 0.50 mol/L, the density of the obtained polymer film is about 0.0045 $nm^{-1}$. That is, according to one embodiment, it can be seen that a high-density polymer film is obtained compared to that in the related art.

Further, FIG. 5 is a graph showing the relationship between the concentration of MPC in an aqueous treatment solution and the weight wear rate of a formed polymer film in examples and comparative examples using the UHMWPE as a base member. From FIG. 5, it can be seen that a polymer film exhibiting a weight wear rate equal to or higher than that of the related art can be obtained when an aqueous treatment solution containing 0.20 mol/L or more and less than 0.50 mol/L of MPC and a water-soluble inorganic salt is used. As described above, it is surprising that the sliding member for an artificial joint exhibiting wear resistance equal to or higher than that of the related art can be obtained even though the aqueous treatment solution having the MPC concentration lower than that of the related art is used.

In particular, from FIGS. 4 and 5, it can be seen that when an aqueous treatment solution containing 0.20 mol/L or more and 0.30 mol/L or less of MPC and a water-soluble inorganic salt is used, a polymer film having a density of 0.007 $nm^{-1}$ or more can be obtained and exhibits excellent wear resistance.

FIG. 6 is a graph showing the relationship between the concentration of MPC in an aqueous treatment solution and the density (ester index/film thickness) of a formed polymer film in examples and comparative examples using the PEEK as a base member. From FIG. 6, it can be seen that a high-density PMPC layer can be obtained when an aqueous treatment solution containing 0.20 mol/L or more and less than 0.50 mol/L of MPC and a water-soluble inorganic salt is used. In particular, when an aqueous treatment solution containing 0.20 mol/L or more and 0.30 mol/L or less of MPC and a water-soluble inorganic salt is used, a polymer film having a density of 0.0005 $nm^{-1}$ or more can be obtained. Compared to this, for example, when the MPC concentration is 0.50 mol/L, the density of the obtained polymer film is about 0.0004 $nm^{-1}$. That is, according to one embodiment, even when the PEEK is used as a base member, it can be seen that a high-density polymer film is obtained compared to that in the related art.

Industrial Applicability

One aspect of the present disclosure is available for manufacturing an artificial joint.

Reference Signs List

1 Artificial hip joint
10 Acetabular cup (sliding member for an artificial joint)
12 Cup base member (base member)

30 Polymer film

## Claims

1. A manufacturing method of a sliding member for an artificial joint, comprising
exposing a base member with ultraviolet rays in a state where the base member is in contact with an aqueous treatment solution containing a compound having 0.20 mol/L or more and less than 0.50 mol/L of phosphorylcholine group and a water-soluble inorganic salt.

2. The manufacturing method of a sliding member for an artificial joint according to claim 1, wherein a concentration of the compound having the phosphorylcholine group is 0.20 mol/L or more and 0.30 mol/L or less.

3. The manufacturing method of a sliding member for an artificial joint according to claim 1 or 2, wherein the concentration of the compound having the phosphorylcholine group is 0.225 mol/L or more and 0.30 mol/L or less.

4. The manufacturing method of a sliding member for an artificial joint according to any one of claims 1 to 3, wherein the water-soluble inorganic salt is an alkali metal salt or an alkaline earth metal salt.

5. The manufacturing method of a sliding member for an artificial joint according to claim 4, wherein the alkali metal salt is one or more selected from a sodium salt, a potassium salt, a lithium salt, and a cesium salt.

6. The manufacturing method of a sliding member for an artificial joint according to any one of claims 1 to 5, further comprising
heating the base member.

7. A sliding member for an artificial joint, comprising:

a base member comprising a compound having a methylene group; and
a polymer film that is formed on at least a portion of a surface of the base member and comprises a polymer chain in which a compound having a phosphorylcholine group is polymerized, wherein
the following formula (1) is satisfied:

$$\text{(peak intensity of phosphoric acid group/peak intensity of methylene group in infrared spectral analysis spectrum)/film thickness of polymer film} \geq 0.007\ \text{nm}^{-1} \ldots (1).$$

8. A sliding member for an artificial joint, comprising:

a base member comprising a compound having a benzene ring; and
a polymer film that is formed on at least a portion of a surface of the base member and comprises a polymer chain in which a compound having an ester group and a phosphorylcholine group is polymerized, wherein
the following formula (2) is satisfied:

$$\text{(peak intensity of ester group/peak intensity of benzene ring in infrared spectral analysis spectrum)/film thickness of polymer film} \geq 0.0005\ \text{nm}^{-1} \ldots (2).$$

9. The sliding member for an artificial joint according to claim 7 or 8, wherein the polymer film has a film thickness of 100 nm or more.

10. An artificial hip joint, comprising
the sliding member for an artificial joint according to any one of claims 7 to 9.

11. The artificial hip joint according to claim 10, comprising:

an acetabular cup comprising the base member and the polymer film; and
a femoral head disposed in the acetabular cup.

12. The artificial hip joint according to claim 11, wherein the polymer film is disposed only on the acetabular cup of the acetabular cup and the femoral head.

13. The artificial hip joint according to claim 11, wherein the polymer film is disposed on both the acetabular cup and the femoral head.

14. The artificial hip joint according to any one of claims 11 to 13, wherein the acetabular cup has a contact surface configured to contact the femoral head, and
the polymer film is disposed on the contact surface.

15. The artificial hip joint according to any one of claims 11 to 14, wherein the femoral head is formed of a material different from that of the base member of the acetabular cup.

16. The artificial hip joint according to any one of claims 11 to 15, wherein the femoral head is formed of a polymer, ceramic, or metal material.

17. The artificial hip joint according to any one of claims 11 to 16, wherein surfaces of the acetabular cup have a first region and a second region having the polymer film thicker than the first region.

18. The artificial hip joint according to claim 17, wherein the first region is located on an outer surface of the surfaces, and the second region is located on an inner surface configured to contact the femoral head of the surfaces.

19. The artificial hip joint according to claim 17 or 18, wherein the first region is located on an edge portion located between an outer surface and an inner surface of the surfaces, the inner surface being configured to contact the femoral head, and
the second region is located on the inner surface.

20. The artificial hip joint according to any one of claims 11 to 19, wherein the acetabular cup has a contact surface configured to contact the femoral head, and
the polymer film is disposed only on the contact surface.

21. The artificial hip joint according to any one of claims 11 to 20, wherein the acetabular cup has a first contact surface configured to contact the femoral head, and
the femoral head has a second contact surface configured to contact the acetabular cup and having a surface roughness less than that of the first contact surface.

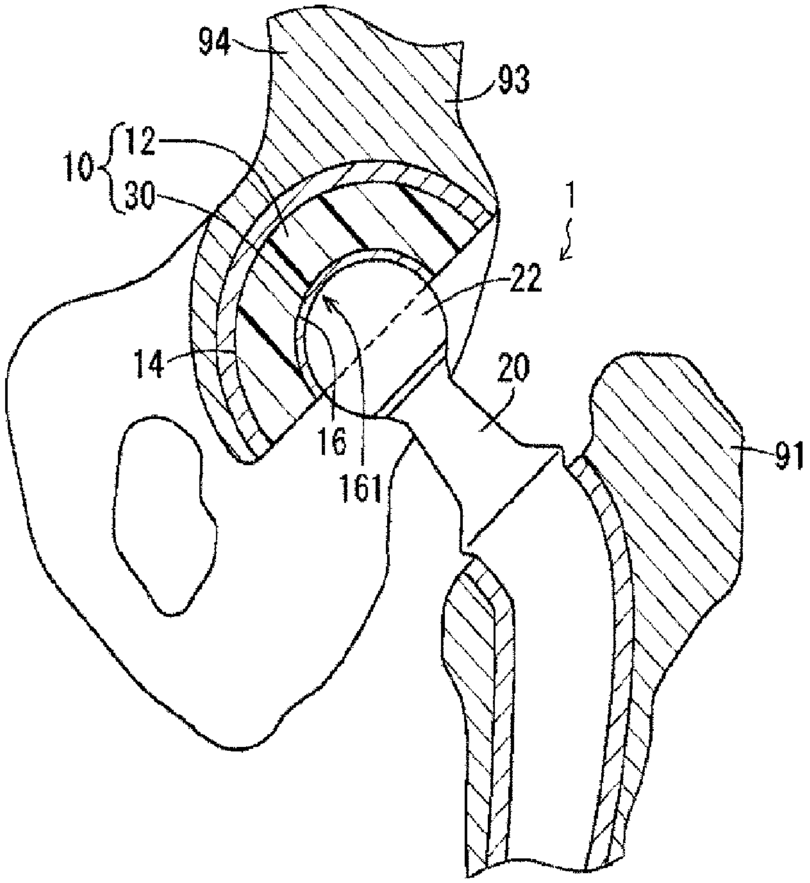


FIG. 1

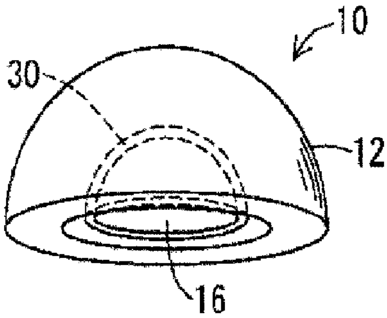


FIG. 2

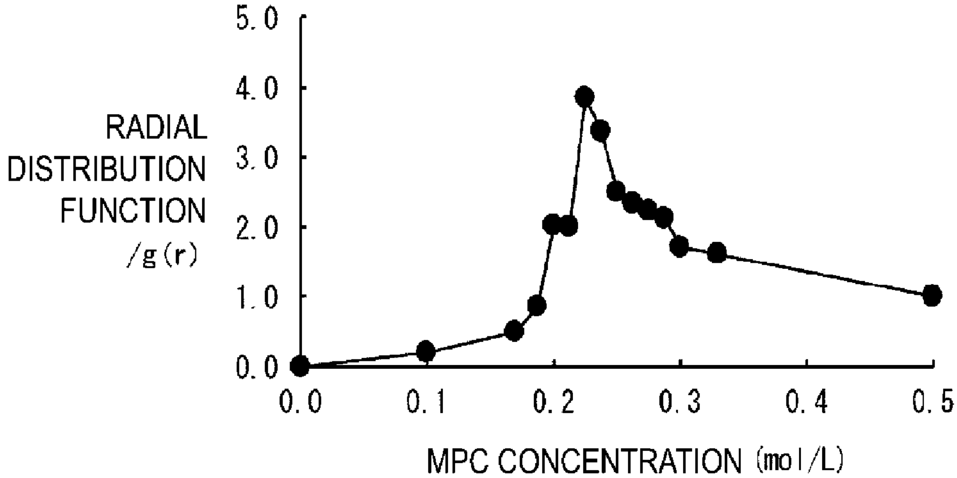
RADIAL
DISTRIBUTION
FUNCTION
/g(r)
5.0
4.0
3.0
2.0
1.0
0.0
0.0
0.1
0.2
0.3
0.4
0.5
MPC CONCENTRATION (mol/L)

FIG. 3

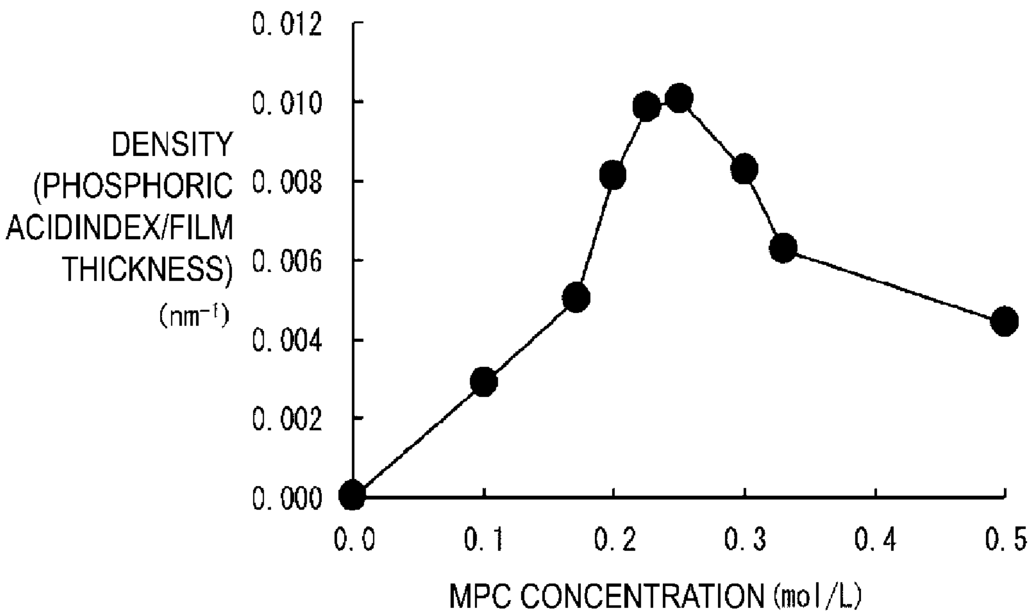
DENSITY (PHOSPHORIC ACIDINDEX/FILM THICKNESS) (nm⁻¹)
0.012
0.010
0.008
0.006
0.004
0.002
0.000
0.0
0.1
0.2
0.3
0.4
0.5
MPC CONCENTRATION (mol/L)

FIG. 4

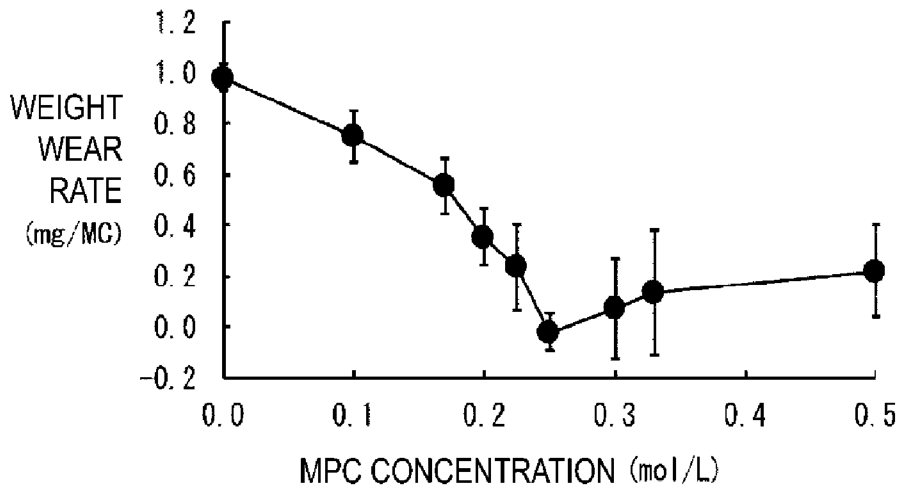
WEIGHT
WEAR
RATE
(mg/MC)
1.2
1.0
0.8
0.6
0.4
0.2
0.0
-0.2
0.0
0.1
0.2
0.3
0.4
0.5
MPC CONCENTRATION (mol/L)

FIG. 5

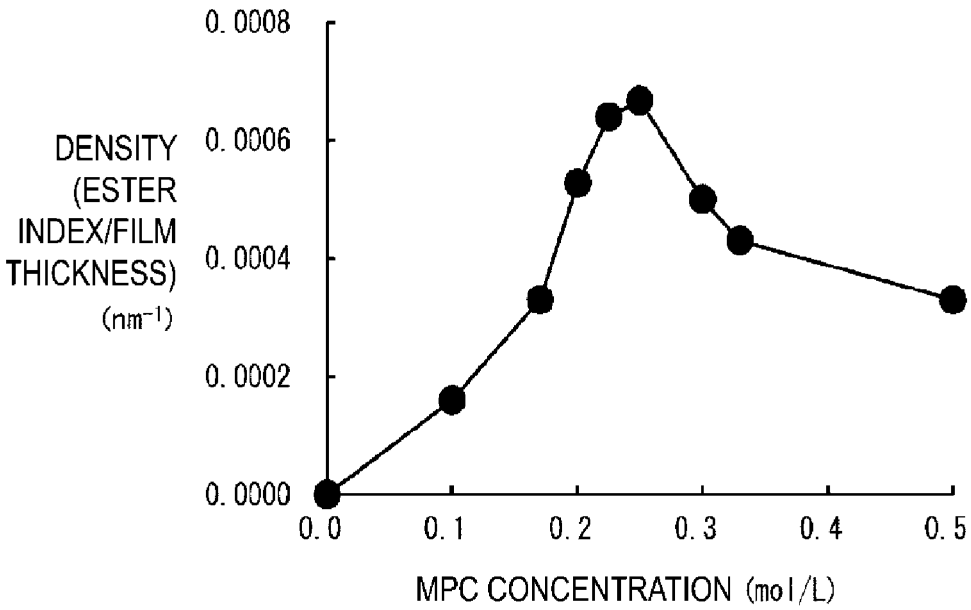
0.0008
0.0006
0.0004
0.0002
0.0000
DENSITY (ESTER INDEX/FILM THICKNESS) ($nm^{-1}$)
0.0
0.1
0.2
0.3
0.4
0.5
MPC CONCENTRATION (mol/L)

FIG. 6

**INTERNATIONAL SEARCH REPORT**

International application No.
PCT/JP2021/000620

A. CLASSIFICATION OF SUBJECT MATTER
A61L 27/34(2006.01)i; A61F 2/30(2006.01)i; A61L 27/04(2006.01)i; A61L 27/10(2006.01)i; A61L 27/14(2006.01)i; A61L 27/16(2006.01)i; A61L 27/18(2006.01)i; A61L 27/32(2006.01)i; A61L 27/36(2006.01)i; A61L 27/50(2006.01)i
FI: A61L27/34; A61L27/36 420; A61L27/32; A61L27/16; A61L27/14; A61L27/10; A61L27/04; A61L27/50; A61L27/18; A61F2/30
According to International Patent Classification (IPC) or to both national classification and IPC

B. FIELDS SEARCHED
Minimum documentation searched (classification system followed by classification symbols)
A61L27/34; A61F2/30; A61L27/04; A61L27/10; A61L27/14; A61L27/16; A61L27/18; A61L27/32; A61L27/36; A61L27/50

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2014-124502 A (KYOCERA MEDICAL CORPORATION) 07 July 2014 (2014-07-07) claims, paragraphs [0008], [0089]-[0093], [0100]-[0106], fig. 6(b), (c) | 7, 9-21<br>1-6<br>8 |
| X<br>A | JP 2014-4352 A (KYOCERA MEDICAL CORPORATION) 16 January 2014 (2014-01-16) claims, paragraphs [0001], [0008], [0084], [0118]-[0124], [0132]-[0138], fig. 2, 19, 20, table 6 | 7, 9-21<br>1-6, 8 |
| X<br>Y<br>A | WO 2011/021642 A1 (JAPAN MEDICAL MATERIALS CORPORATION) 24 February 2011 (2011-02-24) claims, paragraphs [0012]-[0013], [0050]-[0070], examples 1, 2, 4, 5, fig. 1, 4 | 8, 10-21<br>1-6, 8-21<br>7 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 March 2021 (23.03.2021) | 06 April 2021 (06.04.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/000620

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | SHIOJIMA, T. et al., "High-efficiency preparation of poly(2-methacryloyloxyethyl phosphorylcholine) grafting layer on poly(ether ether ketone) by photoinduced and self-initiated graft polymerization in an aqueous solution in the presence of inorganic salt additives", Acta Biomaterialia, 2016, vol. 40, pp. 38-45, abstract, page 39, left column, lines 30-36, fig. 5-8 | 1-6, 8-21<br>7 |
| Y<br>A | JP 2017-144191 A (KYOCERA CORP.) 24 August 2017 (2017-08-24) claims, paragraphs [0027], [0046]-[0048], [0070], [0103], [0106], [0112], fig. 3-5 | 1-6, 8-21<br>7 |

Form PCT/ISA/210 (continuation of second sheet) (January 2015)

**INTERNATIONAL SEARCH REPORT**
Information on patent family members

International application No.
PCT/JP2021/000620

| Patent Documents referred in the Report | Publication Date | Patent Family | Publication Date |
|---|---|---|---|
| JP 2014-124502 A | 07 Jul. 2014 | US 2015/0141545 A1 claims, paragraphs [0258]-[0284], fig. 23B, 23C<br>EP 2856978 A1<br>CN 104349744 A | |
| JP 2014-4352 A | 16 Jan. 2014 | US 2015/0141545 A1 claims, paragraphs [0001], [0010], [0152]-[0153], [0203]-[0214], [0223]-[0233], fig. 2, 19, 20, table 6<br>EP 2856978 A1<br>CN 104349744 A | |
| WO 2011/021642 A1 | 24 Feb. 2011 | US 2012/0197407 A1 claims, paragraphs [0013], [0062]-[0082], examples 1, 2, 4, 5, fig. 1, 4<br>EP 2468311 A1 | |
| JP 2017-144191 A | 24 Aug. 2017 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ISHIHARA et al.** *Polymer Journal,* 1990, vol. 22, 355